# EUROPEAN PATENT APPLICATION

(11) **EP 1 803 705 A1**
(43) Date of publication of application: **04.07.2007**
(21) Application number: 06713044.3
(22) Date of filing: 30.01.2006
(51) Int. Cl.: C07C 213/02, C07C 217/90, C07B 61/00

(54) **AROMATIC DIAMINE AND PROCESS FOR PRODUCING THE SAME**

(30) Priority: 09.02.2005 JP 2005032840
(71) Applicant: JFE Chemical Corporation, Tokyo 111-0051 (JP)
(72) Inventor: MORI, Hiroaki, Taito-ku, Tokyo, 1110051 (JP)
(74) Representative: HOFFMANN EITLE
(86) International application number: PCT/JP2006/301901
(87) International publication number: WO 2006/085493

(57) **Abstract**

The aromatic diamine is represented by the formula I, 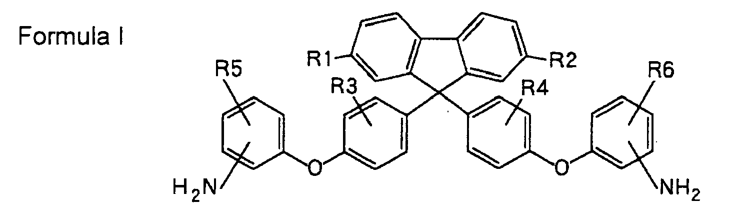 where, R1 to R6 are each independently a hydrogen, an alkyl group, or an aryl group, and they may be the same with or different from each other.

The aromatic diamine is useful as a monomer of polyimide, polyamide, polyamideimide, and the like, or as a curing agent for resins such as polyurethane and epoxy.

## Description

### TECHNICAL FIELD

The present invention relates to a novel aromatic diamine, specifically relates to an aromatic diamine which is useful as a monomer of polymers such as polyimide, polyamide, and polyamideimide, or as a curing agent for resins such as polyurethane and epoxy, and to a method for manufacturing the aromatic diamine.

### BACKGROUND ART

It is known that polyimide and polyamide resins have high heat resistance, mechanical characteristics, resistance to chemicals, and other excellent characteristics.

In recent years, there have shown rapid developments in the electronics equipment field using these resins, in terms of miniaturization of products, increased packaging density of devices, increased communication speed, and the like. In these current developments of use fields, polyimide and polyamide resins are requested not only to improve the above characteristics but also to add new characteristics. For polyimide resins, as an example, there are also requested to have electric characteristics (low dielectric property and the like), low water-absorption, optical characteristics (transparency, high refractive index, and low birefringence), and the like.

To answer these requirements, there have been developed novel diamines as a suitable monomer of polyimide and polyamide resins.

For example, compounds represented by the formula II are disclosed in Japanese Patent Laid-Open Nos. 1-238568, 3-90052, 3-167163, and 7-278069. 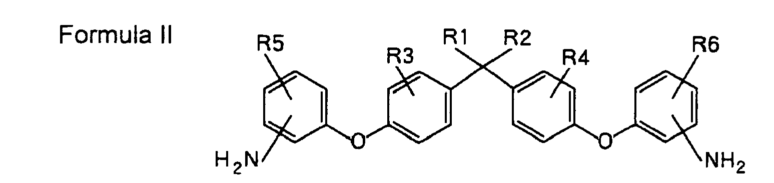 where, R3 to R6 are each an alkyl group, a phenyl group, an alkoxy group, a halogen, or the like, and R1 and R2 are each a hydrogen, an alkyl group, a phenyl group, an alkyl halide group, or the like.

According to these patent publications, those aromatic diamines are useful as a monomer of polyimide, polyamide, polyamideimide, and the like, or as a curing agent for resins such as polyurethane and epoxy, and contribute to the improvement of heat resistance, resistance to water, resistance to chemicals, mechanical characteristics, and electric characteristics.

The above aromatic diamines, however, not necessarily satisfy the requirements of ever-increasing level of resin characteristics in the optical material field and the electronics material field.

The present invention has been derived to answer the above requirements, and an object of the present invention is to provide a novel aromatic diamine which is useful as a monomer of polyimide, polyamide, polyamideimide, and the like, or as a curing agent for resins such as polyurethane and epoxy, and to provide a method for manufacturing the aromatic diamine.

### DISCLOSURE OF THE INVENTION

The present invention is an aromatic diamine represented by the formula I, 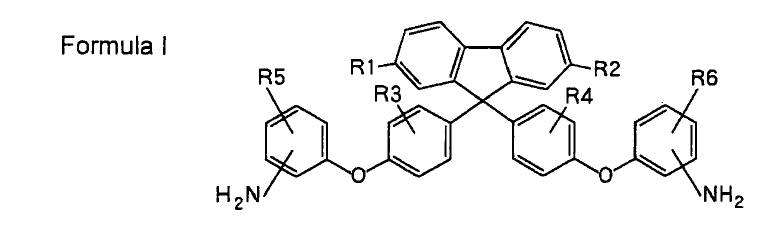 where, R1 to R6 are each independently a hydrogen, an alkyl group, or an aryl group, and they may be the same with or different from each other.

The present invention is also a method for manufacturing aromatic diamine represented by the formula I, which method has: the first step of forming an ether linkage by coupling a fluorenylidenebisphenol compound with an aromatic halonitro compound in the presence of at least one catalyst selected from the group consisting of alkaline compounds, copper, and copper compounds, thus obtaining a corresponding dinitro compound; and a second step of reducing the dinitro compound, 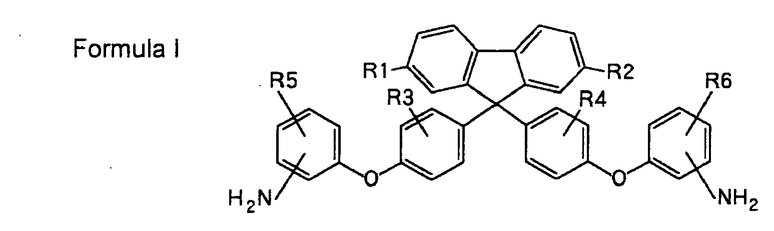 where, R1 to R6 are each independently a hydrogen, an alkyl group, or an aryl group, and they may be the same with or different from each other.

### BEST MODE FOR CARRYING OUT THE INVENTION

The inventor of the present invention carried out detail study to solve the above problems, and found a novel aromatic diamine derived from a fluorenylidenebisphenol compound, and has perfected the present invention.

The present invention is described in detail in the following referring to the preferred embodiments.

Regarding the aromatic diamine according to the present invention, R1 to R6 in the formula I are each a hydrogen, an alkyl group, or aryl group, having 1 to 5 carbon atoms, (R1 to R6 may be the same with or different from each other). Preferably the aromatic diamine is the one in which R1 to R6 are each a hydrogen, a methyl group, or a phenyl group, (R1 to R6 may be the same with or different from each other).

Examples of the aromatic diamine are 9,9-bis[4-(4-aminophenoxy)phenyl]fluorene, 9,9-bis[4-(4-amino-3-methylphenoxy)phenyl]fluorene, 9,9-bis[4-(4-amino-3-ethylphenoxy)phenyl]fluorene, 9,9-bis[4-(4-amino-3-isopropylphenoxy)phenyl]fluorene, 9,9-bis[4-(4-amino-3-phenylphenoxy)phenyl]fluorene, 9,9-bis[4-(4-amino-3-naphthylphenoxy)phenyl]fluorene, 9,9-bis[4-(4-amino-3-methylphenoxy)-3-methylphenyl]fluorene, 9,9-bis[4-(4-amino-3-methylphenoxy)-3-phenylphenyl]fluorene, and 2,7-dimethyl-9,9-bis[4-(4-aminophenoxy)phenyl]fluorene. However, the aromatic diamine is not limited to those given above.

The aromatic diamine according to the present invention has a fluorene skeleton. The fluorene nucleus has properties of bulky and low polarity. As a result, the polymers such as polyamide and polyimide derived from the aromatic diamine of the present invention have a fluorene nucleus in the main chain, which gives an expectation to provide excellent mechanical strength and heat resistance, and further to provide high resistance to chemicals and low dielectric constant. It is a common understanding that the increased dielectric constant of polyimide is caused by the imide group having a polar structure. To this point, when the aromatic diamine according to the present invention is used as the monomer, a low polarity and bulky fluorene nucleus is introduced into the main chain of the polymer, which dilutes the concentration of imide group in the polymer, thereby resulting in decreasing the dielectric constant. Furthermore, as the characteristics of other compounds having the fluorene nucleus suggests, the derivatives of the aromatic diamine of the present invention are expected to give high transparency and low birefringence.

The aromatic diamine according to the present invention can be obtained through the first step of bringing a fluorenylidenebisphenol compound to react with an aromatic halonitro compound in the presence of at least one catalyst selected from the group consisting of alkaline compounds, copper, and copper compounds, thus obtaining a corresponding dinitro compound, and a second step of reducing the dinitro compound.

For easy understanding of the present invention, an example of the reaction is described below using typical compounds.

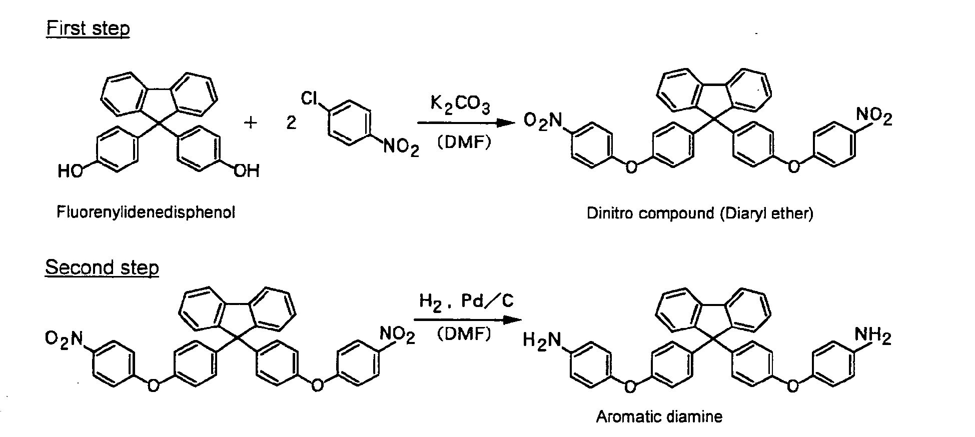

According to the above example, the first step is to bring a fluorenylidenebisphenol compound to couple with an aromatic halonitro compound in the presence of an alkaline carbonate, thus to obtain a corresponding aromatic dinitro compound, and the succeeding second step is to treat the obtained dinitro compound by catalytic reduction with hydrogen using a palladium-carbon catalyst in dimethylformamide (DMF) as an aprotic polar solvent, thereby obtaining a corresponding aromatic diamine.

To obtain the aromatic diamine according to the present invention, the raw materials for the first step may adopt a fluorenylidenebisphenol compound represented by the formula III and an aromatic halonitro compound represented by the formula IV, corresponding to the building blocks of the aromatic diamine.

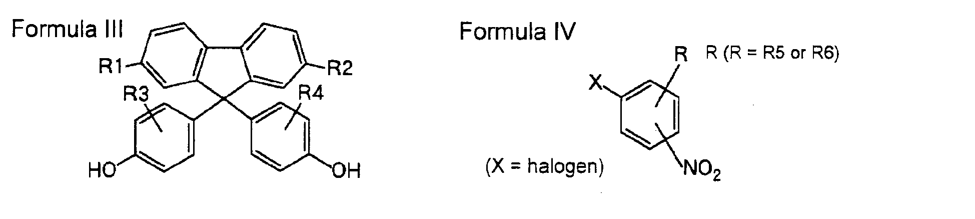

The fluorenylidenebisphenol compound given by the formula III includes the following-given compounds corresponding to the formula I: 9,9-bis(4-hydroxyphenyl)fluorene, 9,9-bis(4-hydroxy-3-methylphenyl)fluorene, 9,9-bis(4-hydroxy-3-isopropylphenyl)fluorene, 9,9-bis(4-hydroxy-3-phenylphenyl)fluorene, 9,9-bis(3-aryl-4-hydroxyphenyl)fluorene, 9,9-bis(4-hydroxy-3-naphthylphenyl)fluorene, 2,7-dimethyl-9,9-bis(4-hydroxyphenyl)fluorene, 2,7-dimethyl-9,9-bis(4-hydroxy-3-methylphenyl)fluorene, and 2,7-dimethyl-9,9-bis(4-hydroxy-3-phenylphenyl)fluorene. Those fluorenylidenebisphenol compounds are easily obtained by a known method of dehydration condensation of corresponding fluorenone or 9, 9-dihalofluorene with phenols in the presence of an acidic catalyst such as sulfuric acid.

The aromatic halonitro compound given in the formula IV includes the following-given compounds corresponding to the formula I: 4-chloronitrobenzene, 4-bromonitrobenzene, 3-chloronitrobenzene, 2-chloronitrobenzene, 2-chloro-3-nitrotoluene, 2-chloro-4-nitrotoluene, 2-chloro-6-nitrotoluene, 4-chloro-2-nitrotoluene, and 4-chloro-3-nitrotoluene.

The use amount of the aromatic halonitro compound is 2 times or more by mole, preferably 2.2 to 2.5 times by mole, to 1 mole of the fluorenylidenebisphenol compound.

The coexisting alkaline compound may be an alkaline carbonate such as sodium carbonate and/or potassium carbonate, an alkaline hydroxide such as sodium hydroxide and/or potassium hydroxide, and a mixture of them. As of these, alkaline carbonate is preferred.

Although the reaction solvent is not an essential component, a preferable solvent includes an aprotic polar solvent such as dimethylformamide, dimethylacetamide, dimethylsulfoxide, sulfolane, N-methylpyrrolidone, and 1,3-dimethyl-2-imidazolidinone. These reaction solvents are preferably used in an amount of 1 to 50 times by mass to the mass of the fluorenylidenebisphenol compound.

An applicable catalyst for the first step includes a copper compound such as copper powder, copper (II) oxide, copper (II) iodide, and copper (II) acetate, and a mixture of them. Those catalysts are used in a range from 0.1 to 100% by mass, preferably 0 . 5 to 10% by mass, to 100% by mass of fluorenylidenebisphenol. If, however, a highly reactive aromatic halonitro compound is used, a side reaction likely occurs, though the main reaction smoothly proceeds. As a result, the purification of the target aromatic diamine by recrystallization becomes extremely difficult. Therefore, if a highly reactive aromatic halonitro compound is used, it is preferred to conduct the reaction of first step without using copper or copper compound as the catalyst. With that procedure, a highly selective diaryl etherification reaction at a degree not requiring purification operation can be conducted. That is, by bringing a fluorenylidenebisphenol to react with an aromatic chloronitro compound in the presence of an alkaline compound, a dinitro compound having a diaryl ether structure is obtained.

In the first step, a fluorenylidenebisphenol compound, an aromatic halonitro compound, potassium carbonate, toluene, and dimethylformamide are poured in a vessel equipped with a stirrer, a thermometer, a Dean-Stalk trap, and a reflux condenser. While keeping the system in nitrogen atmosphere, the contents are heated to 110°C, and the generating water is distilled out as an azeotrope with toluene. After completing the water distillation, the temperature of the system is increased to 150°C to let the contents react at the temperature for 3 hours. After completing the reaction, the reaction product mixture is cooled to room temperature. The reaction product mixture is then charged into water to deposit crystals, which crystals are separated by filtration. Thus prepared crude crystals are rinsed with water to remove inorganic salt. The water-rinsed crystals are further rinsed with methanol, followed by drying to obtain an organic dinitro compound.

In the succeeding second step, the aromatic dinitro compound prepared in the first step is reduced. Since dinitro compounds are very slightly soluble in solvent such as alcohol which is a common solvent, the reaction is difficult to proceed under a typical reducing condition. Accordingly, it is preferable to use an aprotic polar solvent from the point of advantage in terms of solubility, to some degree, and of inert to reaction. Similar to the first step, applicable aprotic polar solvent includes dimethylformamide, dimethylacetamide, dimethylsulfoxide, sulfolane, N-methylpyrrolidone, and 1,3-dimethyl-2-imidazolidinone. These reaction solvents are preferably used in an amount of 1 to 50 times by mass to the mass of the aromatic dinitro compound. The aromatic dinitro compound is not requested to fully dissolve during the reaction. That is, since the aromatic diamine formed in the course of reaction has sufficient solubility in aprotic polar solvent, the generated aromatic diamine after completing the reaction is fully dissolved in the aprotic polar solvent, thus the reaction product mixture after completing the reaction becomes a homogeneous solution except for the catalyst.

The second step is conducted by a known nitro-group reduction. Specifically, it is preferred to adopt catalytic reduction process using molecular hydrogen in the presence of a catalyst.

Applicable catalyst for the reaction includes a metal catalyst such as nickel, Raney nickel, and platinum, and a catalyst prepared by carrying those metals on supports such as active carbon and alumina. Those supported catalysts are used in an amount ranging from 0.1 to 100% by mass, preferably from 0.5 to 30% by mass, to 100% by mass of the dinitro compound. The reaction temperature is in a range from 20°C to 300°C, preferably from 100°C to 200°C. The hydrogen pressure is in a range from atmospheric pressure to 5 MPa, preferably from 0.3 to 1 MPa.

The catalytic reduction is conducted by adding a dinitro compound, a catalyst, and a solvent, at a specified temperature and pressure until the decrease in the hydrogen pressure stops. After completing the reaction, the solid catalyst is removed from the reaction product mixture by filtration. The filtrate is charged into water to collect the deposited solid by filtration. Thus collected crude solid is rinsed with water, and is purified by recrystallization or the like, at need, to obtain the aromatic diamine of the present invention.

### EXAMPLES

The present invention is described in further detail in the following referring to the examples. The present invention, however, is not limited to these examples. In the following description, "%" is weight basis.

### [Example 1]

To a two-liter four-neck flask equipped with a stirrer, a thermometer, a Dean-Stalk trap, and a reflux condenser, there were poured 150 g (0.428 mole) of 9,9-bis(4-hydroxyphenyl)fluorene, 149 g (0.946 mole) of 4-chloronitrobenzene, 130 g (0.943 mole) of potassium carbonate, 50 g of toluene, and 750 g of dimethylformamide. While agitating the contents and keeping the system in a nitrogen atmosphere, the system was heated to 110°C, thus the generated water was distilled out as an azeotrope with toluene. After completing the water distillation, the temperature of the system was increased to 150°C, and the reaction was continued at the temperature for 3 hours. After completing the reaction, the reaction product mixture was cooled to room temperature, which was then charged into 1500 g of water. The deposited crystals were collected by filtration. Thus collected crude crystals were rinsed with 1000 g of water, followed by filtering to remove the residual inorganic salt. The crystals were again rinsed with water and filtered. The crystals (in wet state) were then rinsed with 1000 g of methanol and filtered. After repeated the methanol-rinsing and drying once more, the crystals were filtered and dried to obtain 254 g of corresponding aromatic dinitro compound. The yield of the dinitro compound was 96.2% on the basis of 9,9-bis(4-hydroxyphenyl)fluorene.

A 164 g (0.266 mole) aliquot of thus obtained dinitro compound was put in a one-liter autoclave together with 8.2 g of palladium-carbon (supporting the palladium by 5%), and 600 g of dimethylformamide. The system was brought to nitrogen atmosphere. Then the agitation began, and the system temperature was increased to 150°C. Hydrogen gas was injected to the system to raise the internal pressure to 0.5 MPa. The reaction was continued at the temperature until the hydrogen absorption stops. After completing the reaction, the reaction product mixture was cooled, and the catalyst was separated by filtration. The filtrate was charged into 1200 g of water. The deposited crystals were collected by filtration. Thus prepared crude crystals were further rinsed with 800 g of water for two times to fully remove the solvent. The crystals (in wet state) were dried at 50°C under a reduced pressure to obtain 137 g of 9,9-bis[4-(4-aminophenoxy)phenyl]fluorene. The yield of the product was 95.9% on the basis of dinitro compound, and the purity of the product determined by high performance liquid chromatography was 99.1%.

Thus obtained aromatic diamine showed characteristic absorption position of substituent according to infrared absorption spectra and element analysis as follows.
Infrared absorption spectra
Amino group: 3415 cm⁻¹
Ether group: 1231 cm⁻¹
Element analysis (Molecular formula: C₃₇H₂₈N₂O₂)

**Table 1**

| | C (%) | H (%) | N (%) |
|---|---|---|---|
| Observed value | 83.37 | 5.30 | 5.29 |
| Calculated value | 83.41 | 5.31 | 5.26 |

### [Example 2]

To a two-liter four-neck flask equipped with a stirrer, a thermometer, a Dean-Stalk trap, and a reflux condenser, there were poured 150 g (0.428 mole) of 9,9-bis(4-hydroxyphenyl)fluorene, 149 g (0.946 mole) of 2-chloronitrobenzene, 130 g (0.943 mole) of potassium carbonate, 50 g of toluene, and 700 g of dimethylformamide. The same procedure to that of Example 1 was applied to obtain 246 g of corresponding aromatic dinitro compound. The yield of the obtained dinitro compound was 94.2% on the basis of 9,9-bis (4-hydroxyphenyl)fluorene.

A 150 g (0.246 mole) aliquot of thus obtained dinitro compound was reduced using 7.5 g of palladium-carbon (supporting the palladium by 5%) and 550 g of dimethylformamide by the same procedure to that in Example 1, thus obtained 124 g of 9,9-bis[4-(2-aminophenoxy)phenyl]fluorene. The yield of the product was 93.2% on the basis of dinitro compound, and the purity of the product determined by high performance liquid chromatography was 98.5%.

Thus obtained aromatic diamine showed characteristic absorption position of substituent according to infrared absorption spectra and element analysis as follows.
Infrared absorption spectra
Amino group: 3410 cm⁻¹
Ether group: 1222 cm⁻¹
Element analysis (Molecular formula: C₃₇H₂₈N₂O₂)

**Table 2**

| | C (%) | H (%) | N (%) |
|---|---|---|---|
| Observed value | 83.35 | 5.29 | 5.25 |
| Calculated value | 83.41 | 5.31 | 5.26 |

### [Example 3]

To a two-liter four-neck flask equipped with a stirrer, a thermometer, a Dean-Stalk trap, and a reflux condenser, there were poured 160 g (0.423 mole) of 9,9-bis(4-hydroxy-3-methylphenyl)fluorene, 147 g (0.935 mole) of 4-chloronitrobenzene, 129 g (0.935 mole) of potassium carbonate, 50 g of toluene, and 800 g of dimethylformamide. The same procedure to that of Example 1 was applied to obtain 264 g of corresponding aromatic dinitro compound. The yield of the obtained dinitro compound was 96.6% on the basis of 9,9-bis (4-hydroxy-3-methylphenyl)fluorene.

A 150 g (0.232 mole) aliquot of thus obtained dinitro compound was reduced using 7.5 g of palladium-carbon (supporting the palladium by 5%) and 550 g of dimethylformamide by the same procedure to that in Example 1, thus obtained 122 g of 9,9-bis[4-(4-aminophenoxy)-3-methylphenyl]fluorene. The yield of the product was 92.9% on the basis of dinitro compound, and the purity of the product determined by high performance liquid chromatography was 99.0%.

Thus obtained aromatic diamine showed characteristic absorption position of substituent according to infrared absorption spectra and element analysis as follows.
Infrared absorption spectra
Amino group: 3433 cm⁻¹
Ether group: 1228 cm⁻¹
Element analysis (Molecular formula: C₃₉H₃₂N₂O₂)

**Table 3**

| | C (%) | H (%) | N (%) |
|---|---|---|---|
| Observed value | 83.46 | 5.76 | 5.02 |
| Calculated value | 83.52 | 5.77 | 5.00 |

### [Example 4]

To a two-liter four-neck flask equipped with a stirrer, a thermometer, a Dean-Stalk trap, and a reflux condenser, there were poured 150 g (0.296 mole) of 9,9-bis(4-hydroxy-3-phenylphenyl)fluorene, 103 g (0.650 mole) of 2-chloronitrobenzene, 90 g (0.653 mole) of potassium carbonate, 80 g of toluene, and 1000 g of dimethylformamide. The same procedure to that of Example 1 was applied to obtain 227 g of corresponding aromatic dinitro compound. The yield of the obtained dinitro compound was 95.1% on the basis of 9,9-bis(4-hydroxy-3-phenylphenyl)fluorene.

A 100 g (0.130 mole) aliquot of thus obtained dinitro compound was reduced using 5.0 g of palladium-carbon (supporting the palladium by 5%) and 600 g of dimethylformamide by the same procedure to that in Example 1, thus obtained 85 g of 9,9-bis[4-(2-aminophenoxy)-3-phenylphenyl]fluorene. The yield of the product was 93.8% on the basis of dinitro compound, and the purity of the product determined by high performance liquid chromatography was 98.3%.

Thus obtained aromatic diamine showed characteristic absorption position of substituent according to infrared absorption spectra and element analysis as follows.
Infrared absorption spectra
Amino group: 3411 cm⁻¹
Ether group: 1235 cm⁻¹
Element analysis (Molecular formula: C₄₉H₃₆N₂O₂)

**Table 4**

| | C(%) | H (%) | N (%) |
|---|---|---|---|
| Observed value | 85.86 | 5.24 | 4.14 |
| Calculated value | 85.92 | 5.31 | 4.09 |

The present invention easily provides a novel aromatic diamine having a fluorene nucleus. Since the polymers such as polyimide, polyamide, and polyamideimide, which are derived from the aromatic diamine, contain fluorene nucleus in the main chain, they have not only high mechanical strength and heat resistance but also excellent resistance to chemicals and electric characteristic (low dielectric constant). Furthermore, the aromatic diamine is a useful and novel compound also as a curing agent for resins such as polyurethane and epoxy.

### INDUSTRIAL APPLICABILITY

The present invention allows manufacturing a novel aromatic diamine economically. The aromatic diamine is a useful compound as a monomer of polymers such as polyimide, polyamide, and polyamideimide, or as a curing agent for resins such as polyurethane and epoxy. Consequently, the aromatic diamine contributes to varieties of fields in the society as a raw material of products which respond to the requirements in the electronics equipment field, in terms of miniaturization of products, increased packaging density of devices, and increased communication speed.

## Claims

1. An aromatic diamine represented by the formula I, 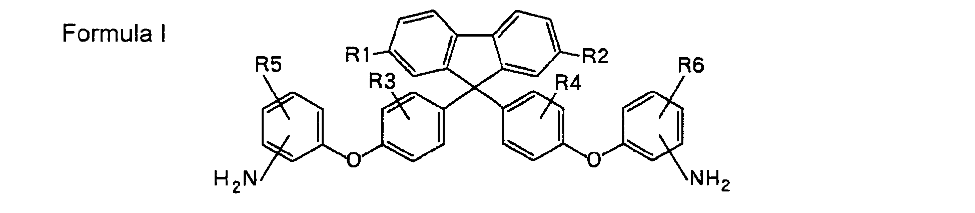 where, R1 to R6 are each independently a hydrogen, an alkyl group, or an aryl group, and they may be the same with or different from each other.

2. A method for manufacturing aromatic diamine represented by the formula I, comprising: a first step of forming an ether linkage by coupling a fluorenylidenebisphenol compound with an aromatic halonitro compound in the presence of at least one catalyst selected from the group consisting of alkaline compounds, copper, and copper compounds, thus obtaining a corresponding dinitro compound; and a second step of reducing the dinitro compound, 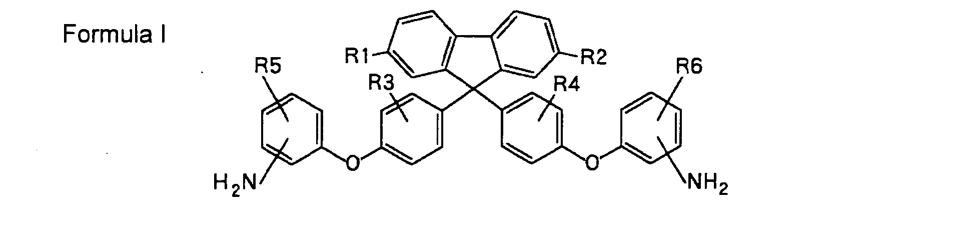 where, R1 to R6 are each independently a hydrogen, an alkyl group, or an aryl group, and they may be the same with or different from each other.
